# EUROPÄISCHE PATENTANMELDUNG

(11) **EP 2 111 891 A1**
(43) Veröffentlichungstag der Anmeldung: **28.10.2009**
(21) Anmeldenummer: 09156054.0
(22) Anmeldetag: 24.03.2009
(51) Int. Cl.: A61N 1/36, A61B 5/0402

(54) **Neurostimulator**

(30) Priorität: 22.04.2008 DE 102008020070
(71) Anmelder: Biotronik CRM Patent AG, 6341 Baar (CH)
(72) Erfinder: Dörr, Thomas, 12437, Berlin (DE); Weiss, Ingo, 12435, Berlin (DE); Neumann, Andreas, 12437, Berlin (DE)
(74) Vertreter: Lindner-Vogt, Karin L.

(57) **Zusammenfassung**

Neurostimulator (20) mit einem Signalgenerator (40) zur Erzeugung von Stimulationsimpulsen, der mit wenigstens einer in das Gehirn eines Patienten implantierbaren Elektrode (10) verbindbar oder verbunden ist, einer Signalgenerator-Steuereinheit (36), welche mit dem Signalgenerator (40) verbunden ist und dazu ausgebildet ist, die Abgabe von Stimulationsimpulsen zu steuern und bei Bedarf Anpassungen von Signalgenerator-Einstellungen vorzunehmen, einer Vorrichtung zur Bestimmung und Analyse der Herzrate eines Patienten (30, 32, 34), welche mit der Signalgenerator-Steuereinheit (36) verbunden ist, **dadurch gekennzeichnet, dass** die Signalgenerator-Steuereinheit (36) dazu ausgebildet ist, unter Verwendung wenigstens einer von der Vorrichtung zur Bestimmung und Analyse der Herzrate (30, 32, 34) ermittelten Herzraten-abhängigen Kenngröße den Gesundheitszustand eines Patienten und/oder einen Therapieerfolg zu überprüfen und ggf. eine Anpassung der Signalgenerator-Einstellungen vorzunehmen.

## Beschreibung

Die Erfindung betrifft einen implantierbaren Neurostimulator zur temporären Stimulation von Körpergewebe, insbesondere zur Hirnstimulation (Deep Brain Stimulation DBS).

Einsatzgebiet derartiger Instrumente sind vor allem neurologische Erkrankungen, wie z. B. Parkinson oder Depression.

Prinzipiell sind Neurostimulatoren für derartige Anwendungsgebiete aus dem Stand der Technik bekannt, weisen jedoch eine Reihe von Nachteilen auf. Einer dieser Nachteile besteht darin, dass bekannte Neurostimulatoren für derartige Anwendungsgebiete eine Therapie ungeachtet des aktuellen Gesundheitszustandes des Patienten durchführen.

Der vorliegenden Erfindung liegt die Aufgabe zugrunde, wenigstens einige Nachteile, die sich aus dem Stand der Technik ergeben, möglichst zu beseitigen.

Erfindungsgemäß wird diese Aufgabe durch einen implantierbaren Neurostimulator gelöst, der folgende Bestandteile aufweist:
- Einen Signalgenerator zur Erzeugung von Stimulationsimpulsen, der mit wenigstens einer in das Gehirn eines Patienten implantierbaren Elektrode verbindbar oder verbunden ist,
- eine Signalgenerator-Steuereinheit, welche mit dem Signalgenerator verbunden ist und dazu ausgebildet ist, die Abgabe von Stimulationsimpulsen zu steuern und bei Bedarf Anpassungen von Signalgenerator-Einstellungen vorzunehmen und
- eine Vorrichtung zur Bestimmung und Analyse der Herzrate eines Patienten, welche mit der Signalgenerator-Steuereinheit verbunden ist.

Erfindungsgemäß ist die Signalgenerator-Steuereinheit dazu ausgebildet, unter Verwendung wenigstens einer von der Vorrichtung zur Bestimmung und Analyse der Herzrate ermittelten Herzraten-abhängigen Kenngröße den Gesundheitszustand eines Patienten und/oder einen Therapieerfolg zu überprüfen und ggf. eine Anpassung der Signalgenerator-Einstellungen vorzunehmen.

Ein Vorteil des erfindungsgemäßen Neurostimulators liegt darin, dass der akute Gesundheitszustand eines Patienten eine Auswirkung auf die Signalgenerator-Einstellungen und somit auf die Therapie hat. Benötigt der Patient aufgrund seines akuten gesundheitlichen Zustandes z. B. eine geringere Stimulationsamplitude, kann die Leistungsabgabe des Neurostimulators durch Anpassung der Signalgenerator-Einstellungen zurückgefahren werden und die Batterie-Lebensdauer des Neurostimulators verlängert sich.

Vorzugsweise ist dabei der Signalgenerator, die Signalgenerator-Steuereinheit und die Vorrichtung zur Bestimmung und Analyse der Herzrate zusammen mit einer Batterie in einem implantierbaren flüssigkeitsdichten Gehäuse untergebracht.

Erfindungsgemäß verfügt der Neurostimulator über wenigstens einen Anschluss zum Anschließen einer implantierbaren Elektrode oder ist über eine elektrische Leitung fest mit dem Neurostimulator verbunden.

Gemäß einer besonders bevorzugten Ausführungsvariante ist der Neurostimulator dazu ausgebildet, die Herzrate eines Patienten über die Ableitung eines Elektrokardiogramms (EKG) zu erfassen. Das Elektrokardiogramm hat sich als besonders einfache und zuverlässige Methode zur Ermittlung der Herzrate eines Patienten erwiesen.

Gemäß einer alternativen Ausführungsform ist der Neurostimulator ausgebildet, die Herzrate eines Patienten über eine Auswertung des Pulses zu bestimmen.

Soll die Herzrate über die Ableitung eines EKG bestimmt werden, so ist der Neurostimulator vorzugsweise dazu ausgebildet, die Herzrate über die zeitlichen Abstände der R-Zacken zu berechnen.

Mit Herzrate ist die Zeitdauer zwischen zwei Herzschlägen gemeint, also die sogenannte "beat-to-beat" Rate.

Gemäß einer besonders bevorzugten Ausgestaltung der Erfindung ist der Neurostimulator dazu ausgebildet, ein EKG zwischen der mit dem Stimulator verbundenen bzw. verbindbaren Elektrode und dem Gehäuse des Neurostimulators abzuleiten.

Für diesen Zweck ist zumindest ein Teil der Oberfläche des Gehäuses als Sensing-Elektrode ausgelegt.

Zur Ableitung der Herzrate aus dem EKG ist der Neurostimulator vorzugsweise dazu ausgebildet, in einem ersten Schritt das EKG mit einer Frequenz von >= 500 Hz abzutasten und zu digitalisieren. Vorzugsweise ist der Neurostimulator dazu ausgebildet, die Herzrate aus dem digitalisierten EKG über die zeitlichen Abstände der R-Zacken zu berechnen.

Vorzugsweise ist der Neurostimulator dazu ausgebildet, die während eines vorgegebenen Zeitraums (im Folgenden auch Analysezeitraum genannt) berechneten Herzraten zu einer Herzraten-abhängigen Kenngröße weiterzuverarbeiten. Mit Hilfe einer solchen Kenngröße wird erfindungsgemäß der aktuelle Gesundheitszustand ermittelt und/oder es wird der Erfolg einer Therapie überprüft.

Besonders bevorzugt ist der Neurostimulator bzw. genauer gesagt die Vorrichtung zur Bestimmung und Analyse der Herzrate dazu ausgebildet, eine frequenzbereichsabgeleitete Kenngröße der Variabilität der Herzraten (HRV) zu berechnen.

Gemäß einer alternativen Ausführungsvariante ist der Neurostimulator bzw. die Vorrichtung zur Bestimmung und Analyse der Herzrate dazu ausgebildet, eine zeitbereichsabgeleitete Kenngröße der Variabilität der Herzraten zu berechnen.

Aus dem Stand der Technik sind bereits eine Vielzahl von sowohl zeitbereichsabgeleiteten Größen, als auch frequenzbereichs-abgeleiteten Größen bekannt und es sei angemerkt, dass jede dieser Kenngrößen oder auch jede Kombination derartiger Kenngrößen zur Ermittlung des aktuellen Gesundheitszustandes eines Patienten oder zur Überprüfung des Therapieerfolgs grundsätzlich herangezogen werden kann, so dass sich weitere, hier nicht explizit erwähnte vorteilhafte Ausführungsformen der Erfindung ergeben.

Für die Behandlung von Patienten mit starker Depression hat sich jedoch gezeigt, dass ein Neurostimulator, welcher dazu ausgebildet ist, den aktuellen Gesundheitszustand mit Hilfe der HRV-Kenngröße RMSSD (Quadratwurzel des quadratischen Mittelwertes der Summe aller Differenzen zwischen benachbarten NN-Intervallen; höhere Werte weisen auf vermehrte parasympathische Aktivität hin) zu berechnen, besonders wirkungsvoll ist.

Um einer Kenngröße auch eine Klassifizierung eines Gesundheitszustandes durchführen zu können, bzw. eine notwendige Anpassung der Signalgenerator-Einstellungen vornehmen zu können, verfügt der Neurostimulator bevorzugt über eine Speichereinheit, in welcher Vergleichswerte (zum Beispiel in einer Liste) abgespeichert sind.

Mit Hilfe derartiger Vergleichswerte kann für jede Kenngröße ein bestimmter Gesundheitszustand bestimmt werden und/oder es können notwendigen Anpassungen der Signalgenerator-Einstellungen vorgenommen werden.

Um nicht für jede berechnete Kenngröße eine Klassifizierung des Gesundheitszustandes mit Hilfe der Vergleichswerte durchführen zu müssen, ist eine weitere Ausführungsvariante dazu ausgebildet, neben den Vergleichswerten auch die Kenngrößen eines vorangehenden Analysezeitraums abzuspeichern. Bevor eine Klassifizierung eines Gesundheitszustandes anhand der ermittelten Kenngröße durchgeführt wird, ist diese Ausbildungsvariante dazu ausgestaltet, zunächst einen Vergleich der aktuell ermittelten Kenngrößen mit der Kenngröße des vorangehenden Analysezeitraums durchzuführen. Überschreitet die Differenz dieser Kenngrößen einen Schwellwert, so ist der Neurostimulator dazu ausgebildet auf eine darauf folgende Klassifizierung eines Gesundheitszustandes, bzw. auf eine Anpassung der Signalgenerator-Einstellungen zu verzichten.

Gemäß einer speziellen Ausführungsvariante ist der erfindungsgemäße Neurostimulator dazu ausgebildet, die aktuelle Kenngröße nicht mit der Kenngröße eines vorangehenden Analysezeitraums, sondern mit einer Kenngröße zu vergleichen, welche zu einer speziellen Tages- oder Nachtzeit ermittelt und abgespeichert worden ist. Ein Vergleich einer gerade berechneten Kenngröße mit der Kenngröße eines früheren Analysezeitraums kann jedoch immer nur zu einer relativen Bewertung des Gesundheitszustandes dienen und keine qualitative Aussage über den Gesundheitszustand machen. Für eine qualitative Bewertung des Gesundheitszustandes ist immer eine Klassifizierung mit den bereits oben erwähnten Vergleichswerten nötig.

Gemäß einer bevorzugten Ausführungsvariante sind die Vergleichswerte nicht unveränderlich, sondern der Neurostimulator ist dazu ausgebildet, z. B. in regelmäßigen Abständen oder gemäß einer anderen Ausführungsvariante auf Wunsch die Vergleichswerte zu aktualisieren.

Zu diesem Zweck ist gemäß einer weiteren Ausführungsvariante auch eine Telemetrie-Einrichtung für den Neurostimulator vorgesehen, um Änderungen und/oder weiterführende Analysen über ein externes Gerät zu ermöglichen.

Gemäß einer weiteren Ausführungsvariante verfügt der Neurostimulator zusätzlich über ein Akzelerometer, welches dazu ausgebildet ist, die körperliche Aktivität eines Patienten in Form einer Kenngröße zu bestimmen und für die Bestimmung des aktuellen Gesundheitszustandes des Patienten zusätzlich zu der Kenngröße für die HRV-Variabilität heranzuziehen.

Da zum Beispiel die HRV-Variabilität eines ruhenden Patienten gegenüber der HRV-Variabilität eines Sport treibenden Patienten selbst bei gleichem Gesundheitszustand unterschiedlich ist, kann die Bestimmung des aktuellen Gesundheitszustandes unter Verwendung einer Kenngröße für die körperliche Aktivität des Patienten ein genaueres Ergebnis liefern.

In diesem Zusammenhang ist vorgesehen, dass die Steuereinheit dazu ausgebildet ist, entweder den Algorithmus zur Berechnung der Herzraten-abhängigen Kenngröße oder aber den Vergleichswert zur Bestimmung des akuten Gesundheitszustandes entsprechend der ermittelten Kenngröße für die Aktivität des Patienten anzupassen.

Gemäß der bisher beschriebenen Ausführungsvarianten ist der Neurostimulator immer dazu ausgebildet, die notwendigen Anpassungen der Signalgenerator-Einstellungen nach einer Analyse Herzraten-abhängigen Kenngröße vorzunehmen.

Gemäß einer weiteren Ausführungsvariante ist der Neurostimulator jedoch auch dazu ausgebildet, zunächst eine vorgegebene Stimulationstherapie durchzuführen und in einem zweiten Schritt deren Erfolg zu kontrollieren.

Ein für die schon weiter oben beschriebene Depressions-Therapie vorgesehener Neurostimulator ist gemäß einer besonderen Ausführungsform dazu ausgebildet, nacheinander folgende Arbeitsschritte durchzuführen:

Zunächst wird mit der gerade eingestellten Ausgangsamplitude an fünf Tagen der Woche in einem täglich vorbestimmten Zeitfenster die Kenngröße RMSSD bestimmt und abgespeichert. Am 6. Wochentag erfolgt eine RMSSD-Berechnung während der Neurostimulator mit einer erhöhten Amplitude stimuliert. Ist der während dieser Messung berechnete SDANN-Wert deutlich größer als der Mittelwert der zuvor bestimmten RMSSD-Werte, so wird die zuvor eingestellte Stimulationsamplitude als zu gering erachtet und daher die erhöhte Stimulationsamplitude aus dem Test des 6. Tages permanent eingestellt, andernfalls wird die alte Einstellung beibehalten. Anschließend erfolgt am 7. Wochentag eine RMSSD-Berechnung, während der Neurostimulator mit einer reduzierten Amplitude stimuliert. Ist der RMSSD-Wert zuzüglich einer definierten Toleranz im Bereich der Vergleichswerte vom Tag 1-5, so wird die reduzierte Stimulationsamplitude als effektiv erachtet und die dauerhafte Stimulationsamplitude wird entsprechend reduziert. Ist der während dieser Messung berechnete RMSSD-Wert deutlich kleiner als der Mittelwert der zuvor bestimmten RMSSD-Werte, so wird die getestete Stimulationsamplitude als zu gering erachtet und daher der vor dem Test aktive Wert für die Stimulationsamplitude wieder eingestellt.

Weitere Aspekte des erfindungsgemäßen Implantats und verschiedene Ausführungsvarianten sind anhand der Zeichnungen näher erläutert. Es zeigen:
- Fig. 1:: Eine Prinzipskizze für die Implantation des erfindungsgemäßen Neurostimulators im Körper eines Patienten;
- Fig. 2:: ein Blockschaltbild einer Ausführungsvariante des erfindungsgemäßen Neurostimulators mit EKG-Steuerung;
- Fig. 3:: ein Blockschaltbild, welches eine EKG-Signalverarbeitungseinheit und eine Herzraten-Messeinheit detailliert darstellt.

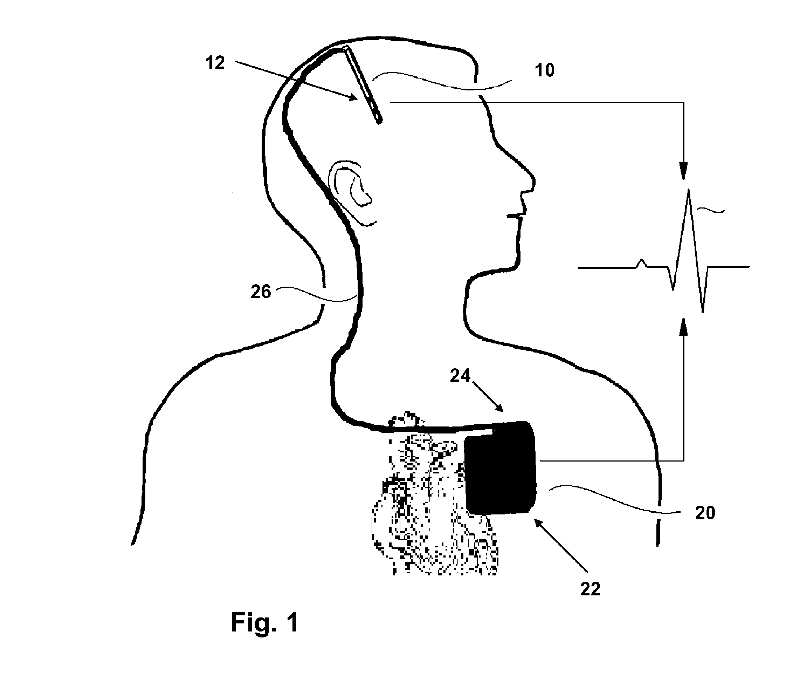

Fig. 1 zeigt einen implantierbaren Neurostimulator 20 mit einem Gehäuse 22 und einem Header 24. Das Gehäuse 22 ist hohl und besitzt eine wenigstens teilweise elektrisch leitende Oberfläche. Typischer Weise besteht das Gehäuse 22 aus einem biokompatiblen Metall wie Titan. In dem Gehäuse 22 befinden sich eine Batterie und elektronische Komponente des Neurostimulators 20. Der Header 24 enthält Anschlussbuchsen, beispielsweise für eine Zuleitung 26.

Eine Elektrode 10 zur tiefen Hirnstimulation (DBS) ist über eine Zuleitung 26 mit wenigstens einer Anschlussbuchse im Header 24 elektrisch verbunden. Die Elektrode 10 weist dabei mehrere Elektrodenpole 12 auf, über die im Behandlungsfall stimulierende Impulse abgegeben werden können.

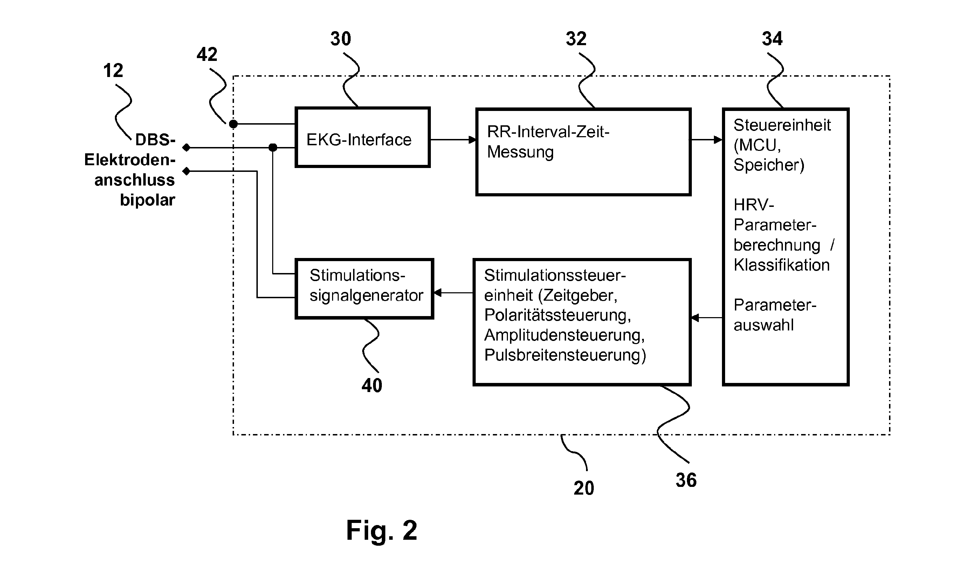

Wenigstens einer der Elektrodenpole 12 bildet zusammen mit dem elektrisch leitenden Abschnitt des Gehäuses ein Elektrodenpaar für das Ableiten eines Elektrokardiogramms (EKG). Hierzu sind wenigstens einer der Elektrodenpole 12 und die leitende Oberfläche des Gehäuses 22 mit einer hier nicht sichtbaren EKG-Signalverarbeitungseinheit 30 im Inneren des Gehäuses 22 des Neurostimulators 20 verbunden (Näheres ist mit Bezug auf Fig. 2 beschrieben).

In der Anwendung ist die mit dem Neurostimulator 20 zu verbindende Elektrode 10 zur tiefen Hirnstimulation derart im Gehirn des Patienten positioniert, dass das Zielgebiet der Stimulation bei der Behandlung der parkinsonschen Erkrankung der Nucleus subthalamicus oder bei essentiellem Tremor der Thalamus ventralis bzw. bei Dystonie der Globus pallidus ist. Für die Behandlung der Depression ist das Zielgebiet der Stimulation der Nucleus accumbens.

Ist der Neurostimulator 20 und die mit ihm verbundene Elektrode 10 im Patienten implantiert, wird entweder kontinuierlich oder temporär ein EKG zwischen dem entsprechenden Therapieort im Gehirn des Patienten und dem Ort, an dem das Gehäuse 22 positioniert ist (z. B. unterhalb der Brustmuskulatur), abgeleitet und in einer hier nicht sichtbaren Herzraten-Analyseeinheit innerhalb des Gehäuses 22 analysiert.

Wie zu Figur 2 noch ausführlicher erläutert wird, erfolgt eine Stimulation der entsprechenden Zielgebiete im Gehirn eines Patienten entweder dann, wenn dies ein gesundheitlicher Zustand des Patienten akut erfordert oder kontinuierlich, entsprechend vorgegebener Signalgenerator-Einstellungen.

Fig. 2 zeigt in einem schematischen Blockschaltbild die elektronischen Komponenten des Neurostimulators 20, die im Inneren des Gehäuses 22 angeordnet sind.

In der hier dargestellten Ausführungsvariante umfasst der Neurostimulator 20 eine EKG-Signalverarbeitungseinheit 30, eine Herzraten-Messeinheit 32 und eine Herzraten-Analyseeinheit 34, welche zusammen die erfindungsgemäße Vorrichtung zur Bestimmung und Analyse der Herzrate repräsentieren. Zusätzlich umfasst der Neurostimulator 20 eine Signalgenerator-Steuereinheit 36 und einen Signalgenerator 40 und ist mit zwei Stimulationselektrodenpolen 12 und einer Sensing-Elektrode 42 verbunden.

Zur Therapiesteuerung bzw. zur Anpassung der Einstellungen des Signalgenerators 40 wird mittels der EKG-Signalverarbeitungseinheit 30 zunächst das EKG des Patienten zwischen einem Pol 12 der Gehirnelektrode 10, und der Sensingelektrode 42 des Neurostimulators abgeleitet.

Die EKG-Signalverarbeitungseinheit 30 stellt dabei der Herzraten-Messeinheit 32 ein Signal zur Verfügung, das jeweils einen Herzschlag (R-Welle) repräsentiert.

Die Herzraten-Messeinheit 32 bestimmt den zeitlichen Abstand zwischen den R-Wellen und stellt diese Information - nämlich die Herzrate - der Herzraten-Analyseeinheit 34 zur Verfügung.

Während eines festgelegten Analysezeitraums erhält die Herzraten-Analyseeinheit 34 auf diese Art und Weise eine Mehrzahl aufeinander folgender Herzraten, aus welchen sie eine oder mehrere Kenngrößen der Herzvariabilität berechnet. Nachdem die Herzraten-Analyseeinheit 34, welche als eine mikroprozessorgesteuerte Baugruppe ausgestaltet ist, eine oder mehrere Kenngrößen der Herzvariabilität wie z.B. SDANN (Standardabweichung des Mittelwertes der NN-Intervalle in allen Fünf-Minuten der gesamten Aufzeichnung) berechnet hat, stellt sie diese der Signalgenerator-Steuereinheit 36 zur Verfügung.

Die Signalgenerator-Steuereinheit umfasst eine hier nicht sichtbare Kurzzeitspeichereinheit und eine ebenfalls nicht sichtbare Langzeitspeichereinheit.

In der Langzeitspeichereinheit ist eine Klassifikationstabelle abgespeichert, in der jeder Kenngröße ein Gesundheitszustand und/oder bestimmte Generatoreinstellungen zugeordnet werden.

In der Kurzzeitspeichereinheit wird jede von der Herzraten-Analyseeinheit 34 erhaltene Kenngröße für die Dauer wenigstens eines Analysezeitraums abgespeichert, um bei Empfang der nächsten Kenngröße abrufbar zu sein.

Um zu Entscheiden, ob eine Anpassung der Einstellungen des Signalgenerators 40 durchgeführt werden muss, wird nämlich in einem ersten Schritt eine gerade ermittelte Kenngröße mit der abgespeicherten Kenngröße des vorangehenden Analysezeitraums verglichen und eine Differenz gebildet.

Überschreitet oder unterscheitet diese Differenz zweier aufeinander folgender Analysezeiträume einen festgelegten Grenzwert, so steht fest, ob eine Anpassung der Signalgenerator-Einstellungen durchgeführt werden muss.

Die Anpassung der Signalgenerator-Einstellungen erfolgt in einem zweiten Schritt, in dem die gerade ermittelte Kenngröße mit Hilfe der im Langzeitspeicher hinterlegten Klassifikationstabelle einem bestimmten Gesundheitszustand und/oder bestimmten Signalgenerator-Einstellungen zugeordnet wird.

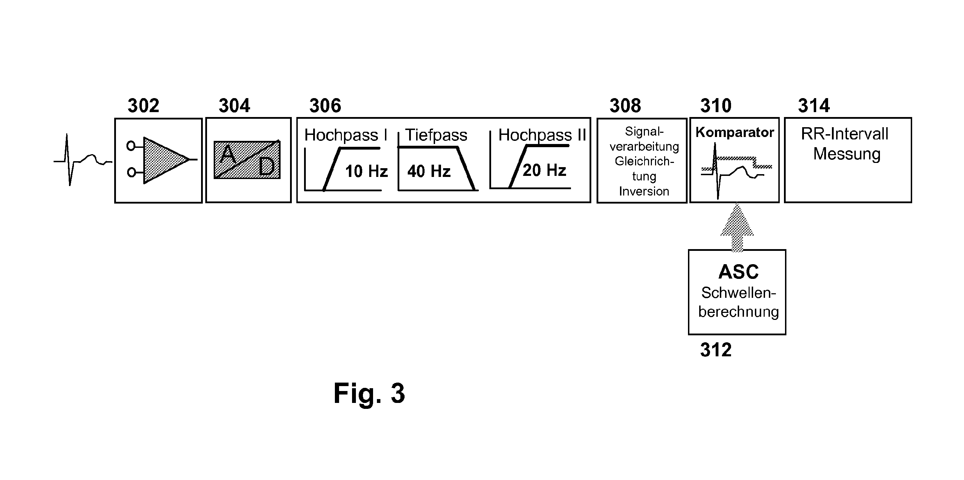

In der Abbildung 3 ist ein detailliertes Blockschaltbild der einzelnen Bauteile der EKG-Signalverarbeitungseinheit 30 und der Herzraten-Messeinheit 32 dargestellt.

Nach einer EKG-Signalverstärkung (302) erfolgt eine Digitalisierung (304) des Eingangssignals mit einer Abtastrate von mindestens 500 Hz, bevorzugt jedoch mehr als 1 KHz. Anschließend erfolgt eine digitale Signalfilterung unter Verwendung programmierbarer 3-stufiger Digitalfilter (306), wobei im Ausführungsbeispiel eine Filterkaskade aus einem ersten Hochpass-, einem Tiefpass- und einem zweiten Hochpassfilter 1. Ordnung vorgesehen ist, so dass die R-Welle als Nutzsignal herausgefiltert wird.

In einer anschließenden Signalkonditionierungsstufe (308) erfolgt eine Halbwellengleichrichtung des gefilterten Signals, wobei eine automatische Umschaltung auf die Halbwelle mit der größten Nutzsignalamplitude optional aktiviert werden kann. Im anschließenden Komparator (310) erfolgt die Erkennung der R-Wellen mittels Schwellwertvergleich.

Die Vergleichsschwellen werden dabei dynamisch an die Nutzsignalamplitude angepasst. Diese Anpassung wird von einer programmierbaren Steuereinheit (312) zur Schwellenbestimmung vorgenommen. In der anschließenden Zeitmesseinheit (314) wird der Abstand der vom Komparator (310) bestimmten R-Wellen mit einer Zeitbasis von kleiner/gleich 10 ms vermessen.

## Patentansprüche

1. Implantierbarer Neurostimulator (20) zur Behandlung von Patienten insbesondere mit parkinsonscher Erkrankung oder Depression mit
- einem Signalgenerator (40) zur Erzeugung von Stimulationsimpulsen, der mit wenigstens einer in das Gehirn eines Patienten implantierbaren Elektrode (10) verbindbar oder verbunden ist,
- einer Signalgenerator-Steuereinheit (36), welche mit dem Signalgenerator (40) verbunden ist und dazu ausgebildet ist, die Abgabe von Stimulationsimpulsen zu steuern und bei Bedarf Anpassungen von Signalgenerator-Einstellungen vorzunehmen,
- einer Vorrichtung zur Bestimmung und Analyse der Herzrate eines Patienten (30, 32, 34), welche mit der Signalgenerator-Steuereinheit (36) verbunden ist,
**dadurch gekennzeichnet, dass** die Signalgenerator-Steuereinheit (36) dazu ausgebildet ist, unter Verwendung wenigstens einer von der Vorrichtung zur Bestimmung und Analyse der Herzrate (30, 32, 34) ermittelten Herzraten-abhängigen Kenngröße den Gesundheitszustand eines Patienten und/oder einen Therapieerfolg zu überprüfen und ggf. eine Anpassung der Signalgenerator-Einstellungen vorzunehmen.

2. Neurostimulator (20) nach Anspruch 1, **dadurch gekennzeichnet, dass** der Signalgenerator (40), die Signalgenerator-Steuereinheit (36) und die Vorrichtung zur Bestimmung und Analyse der Herzrate (30, 32, 34) zusammen mit einer Batterie in einem implantierbaren flüssigkeitsdichten Gehäuse (22) untergebracht sind.

3. Neurostimulator (20) nach einem der vorangehenden Ansprüche, welcher dazu ausgebildet ist, die Herzrate über die Ableitung eines Elektrokardiogramms (EKG) zu bestimmen.

4. Neurostimulator (20) nach Anspruch 3 mit einem wenigstens teilweise elektrisch leitenden Gehäuse, welcher dazu ausgebildet ist, das EKG zwischen einer Gehirnelektrode und dem Gehäuse (22) abzuleiten.

5. Neurostimulator (20) nach einem der Ansprüche 3 oder 4, welcher dazu ausgebildet ist, das abgeleitete EKG mit einer Frequenz von >= 500 Hz abzutasten und zu digitalisieren.

6. Neurostimulator (20) nach einem der vorangehenden Ansprüche, **dadurch gekennzeichnet, dass** die Herzraten-abhängige Kenngröße eine frequenzbereichsabgeleitete Größe der Variabilität der Herzraten (HRV) ist.

7. Neurostimulator (20) nach einem der vorangehenden Ansprüche, **dadurch gekennzeichnet, dass** die Herzraten-abhängigen Kenngröße eine zeitbereichsabgeleitete Größe der Variabilität der Herzraten (HRV) ist.

8. Neurostimulator (20) nach einem der vorangehenden Ansprüche, welcher über eine Speichereinheit verfügt, die mit der Signalgenerator-Steuereinheit (36) verbunden ist und dazu ausgebildet ist, wenigstens eine Herzraten-abhängigen Kenngröße zu speichern.

9. Neurostimulator (20) nach einem der vorangehenden Ansprüche, welcher über eine Speichereinheit verfügt, die mit der Signalgenerator-Steuereinheit (36) verbunden ist, wobei die Signalgenerator-Steuereinheit (36) dazu ausgebildet ist mit Hilfe einer in der Speichereinheit abgespeicherten Klassifikationstabelle, in der einer Reihe von Vergleichswerten bestimmten Gesundheitszuständen und/oder bestimmten Signalgenerator-Einstellungen zugeordnet sind, jeder Kenngröße einen Gesundheitszustand und/oder bestimmte Signalgenerator-Einstellungen zuzuordnen.

10. Neurostimulator (20) nach einem der vorangehenden Ansprüche, welcher dazu ausgebildet ist, einer Kenngröße nur dann einen Gesundheitszustand und/oder bestimmte Signalgenerator-Einstellungen zuzuordnen, wenn die Differenz zwischen einer gerade berechneten Kenngröße und einer zu einem früheren Zeitpunkt berechneten und gespeicherten Kenngröße einen bestimmten Grenzwert über- bzw. unterschreitet.

11. Neurostimulator (20) nach einem der vorangehenden Ansprüche, welcher dazu ausgebildet ist, den Therapieerfolg einer neuen Signalgenerator-Einstellung zu überprüfen, indem
- zunächst die Differenz zwischen einer gerade berechneten Kenngröße und einem Mittelwert mehrerer zu einem früheren Zeitpunkt berechneter und gespeicherter Kenngrößen gebildet wird und
- anschließend diese Differenz mit einem Grenzwert verglichen wird.

12. Neurostimulator (20) nach einem der vorangehenden Ansprüche, welcher über ein Akzelerometer verfügt, das dazu ausgebildet ist, die Aktivität eines Patienten in Form einer Kenngröße zu bestimmen.

13. Neurostimulator (20) nach Anspruch 12, **dadurch gekennzeichnet, dass** die auf die Aktivität eines Patienten bezogene Kenngröße zur Bestimmung des Gesundheitszustandes und/oder zur Anpassung der Signalgenerator-Einstellungen herangezogen wird.
